# EUROPEAN PATENT APPLICATION

(11) **EP 1 059 085 A1**
(43) Date of publication of application: **13.12.2000**
(21) Application number: 99906462.9
(22) Date of filing: 24.02.1999
(51) Int. Cl.: A61K 31/59

(54) **LANGERHANS CELL MIGRATION INHIBITORS**

(30) Priority: 26.02.1998 JP 4475798
(71) Applicant: CHUGAI SEIYAKU KABUSHIKI KAISHA, Tokyo, 115-8543 (JP)
(72) Inventor: KINOSHITA, Shigeru Kyoto Prefectural Uni. of Medi., Kyoto-shi Kyoto 602-0841 (JP)
(74) Representative: VOSSIUS & PARTNER
(86) International application number: JP9900833
(87) International publication number: WO9943330

(57) **Abstract**

The present invention provides a Langerhans cell migration inhibitor comprising an active vitamin D as an active ingredient. The Langerhans cell migration inhibitor is useful for preventing immunoreactive inflammation of the skin or cornea and treating it after it occurs, and has less side effects.

## Description

### FIELD OF THE INVENTION

The present invention relates to Langerhans cell migration inhibitors, and more specifically, Langerhans cell migration inhibitors comprising active vitamin D as an active ingredient.

### PRIOR ART

Langerhans cells take up a foreign antigen into the skin, move to lymphatic ducts, where they become veiled cells to intracellularly partially degrade the antigen, and then move to adjacent lymphatic organs to present the partially degraded antigen to T cells whereby the T cells are differentiated into contact dermatitis-inducing cells. Langerhans cells are also known to migrate from the corneal limbus into the central cornea, where they produce various cytokines to induce immunoreactive inflammation.

Since Langerhans cells are deeply involved in the development stage of immunoreactive inflammation of the skin and cornea as described above, continued limitation of the number of these cells at the entry site of foreign antigens seems to prevent the inflammation at the surrounding sites. Especially, it is important to prevent corneal inflammation, which affects corneal transparency and thus lowers the vision of the patient.

At present, potent drugs such as steroids and cyclospolin A are used to inhibit immunoreaction in the skin or cornea, but these drugs are inappropriate for use before inflammation occurs because of their various side effects. Therefore, there is a demand for development of a drug with less side effects that can be safely used before inflammation occurs.

USP. No. 4,610,478 discloses a composition for topical treatment of dermatitis, comprising active vitamin D, 1-α-hydroxylcholecalciferol or 1-α,25-dihydroxylcholecalciferol.

JP No. 503922/93A (WO91/05537) discloses a method for promoting the cure of wounds, comprising administering a vitamin D compound to the patient.

WO96/29079 discloses an opthalmic composition for treating inflammation in the anterior part of the eyeball, comprising an active vitamin D as an active ingredient.

These publications disclose the antiinflammatory agents with less side effects comprising an active vitamin D instead of steroids and therapies using them, but all of them are directed to a therapy of inflammation already developed and none of them suggest inhibition of migration of Langerhans cells that are deeply involved in the development stage of immunoreactive inflammation.

An object of the present invention is to provide a pharmaceutical composition with less side effects that can prevent immunoreactive inflammation of the skin or cornea and also can cure the inflammation after it occurs.

### SUMMARY OF THE INVENTION

The present invention provides a Langerhans cell migration inhibitor comprising an active vitamin D as an active ingredient. This active vitamin D is preferably active vitamin D₃ or a derivative or analog of vitamin D₃, more preferably Active vitamin D₃, and most preferably calcitriol or 22-oxacalcitriol.

The Langerhans cell migration inhibitor of the present invention can be preferably in the form of an ophthalmic solution, and can be used for the prevention or treatment of ocular inflammation, particularly keratoconjunctivitis as well as the prevention of phlyctenular keratitis or corneal infiltration.

### BRIEF DESCRIPTION OF THE DRAWINGS

FIG. 1 is a graph showing the score of neovascularization in murine corneas in Test example 1. The ordinate indicates the score and the abscissa indicates the number of days after stitching.
FIG. 2 is a graph showing the effect of calcitriol (1α,25(OH)₂D₃) on the proliferation of human corneal epithelial cells in Test example 2.
FIG. 3 is a graph showing the effect of calcitriol on IL-1α production in Test example 2.
FIG. 4 is a graph showing the effect of calcitriol on IL-1β production in Test example 2.
FIG. 5 is a graph showing the effect of calcitriol on IL-8 production in Test example 2.

### DETAILED DESCRIPTION OF THE INVENTION

Examples of inflammation to which Langerhans cell migration inhibitors of the present invention can be applied include inflammations of the surface layer of the cornea or conjunctiva such as allergic keratoconjunctivitis, spring catarrh and diffuse keratoconjunctivitis, and inflammations reaching the corneal stroma such as corneal degeneration, corneal infiltration and phlyctenular keratitis. Especially, they are preferably applied to allergic keratoconjunctivitis.

Examples of active vitamin D which is an active ingredient of the composition of the present invention include cholecalciferol derivatives, cholecalciferol analogs, ergocalciferol derivatives, ergocalciferol analogs and hydrophilic active vitamin D analogs having a hydrophilic group at the side chain. These active vitamins D may be natural or synthesized. Specific examples of the active vitamin D include calcitriol (1α,25-dihydroxyvitamin D₃ (1α,25(OH)₂D₃)), 1α,24-dihydroxyvitamin D₃, alphacalcidol (1α-hydroxyvitamin D₃), calcifedol (25-hydroxyvitamin D₃), 1α,25,26-trihydroxyvitamin D₃, 1β,25-dihydroxyvitamin D₃, 24-homo-1α,25-dihydroxyvitamin D₃, 26-homo-1α,25-dihydroxyvitamin D₃, OCT (22-oxacalcitriol), and calcipotriol. Preferred active vitamins D are vitamin D₃, vitamin D₃ derivatives and vitamin D₃ analogs, specifically, calcitriol and 22-oxacalcitriol.

The Langerhans cell migration inhibitor of the present invention is generally used in the form of a pharmaceutical composition comprising active vitamin D in combination with a pharmaceutical carrier and formulated as a solution or ointment for topical administration. The compositions are preferably formulated as an ophthalmic liniment, especially an ophthalmic solution. Examples of carriers suitable for these formulations include, but not limited to, water, ethanol, ethylene glycol, propylene glycol, polyvinyl alcohol, polyvinyl pyrrolidone, gum arabic, calcium phosphate, alginate, gum tragacanth, gelatin, methylcellulose, talc, magnesium stearate, hyaluronic acid, chondroitin sulfate, collagen, fat and mineral oils. In addition to these carriers, the pharmaceutical compositions of the present invention may contain other additives such as a stabilizer, preservative, isotonizing agent, pH-modifier, antioxidant and colorant. Examples of the stabilizer include, but not limited to, sodium bisulfite, glycerin, sodium edetate, sodium citrate and butyl hydroxyanisole. Example of the preservative include, but not limited to, benzalkonium chloride. Example of the isotonizing agent include, but not limited to, sodium chloride, D-mannitol, glucose and glycerin. Example of the pH-modifier include, but not limited to, phosphates such as monosodium phosphate and sodium hydrogenphosphate, sodium hydroxide and hydrochloric acid. Examples of the antioxidant include, but not limited to, vitamin C.

The pharmaceutical compositions of the present invention can be prepared by methods known in the art in any of accelerated-release, controlled-release and delayed-release dosage forms. The concentration of active vitamin D in the compositions of the present invention depends on the dosage form, but generally ranges from about 0.01 ng/ml to about 1 µg/ml, preferably from about 1 to about 100 ng/ml.

In a preferred embodiment, an ophthalmic solution is prepared by dissolving calcitriol or 22-oxacalcitriol as an active vitamin D in water or ethanol to a final active vitamin D concentration of about 1 to about 100 ng/ml. The pH is typically adjusted to about 5.0-9.0, preferably about 7.0-8.0. When the active vitamin D is less soluble, a solubilizing agent such as Polysorbate 80, propylene glycol, polyvinyl pyrrolidone K30 or Poloxamer 188 may be added. Thus prepared ophthalmic solution is typically applied to the eye, but not limited to, at a dose of one to several drops once to four times per day depending on the condition.

By applying the composition of the present invention to the site at which inflammation is to be prevented, development of an immunoreactive inflammation at that site can be significantly prevented as a result of the inhibition of migration of Langerhans cells that may mediate the development of the inflammation. Even when inflammation has already occurred, it can also be treated. Particularly, an inflammation at the cornea can be treated without lowering the transparency of the cornea.

The following examples further illustrate the present invention, without limiting the same thereto.

### EXAMPLES

### Example 1

A stock solution of active vitamin D₃ (containing 50 mg/ml of 1α-hydroxyvitamin D₃) was diluted 1000-fold with ethanol, and further diluted 100-fold with an ophthalmic physiological buffer to prepare an ophthalmic composition containing 0.5 µg/ml of 1α-hydroxyvitamin D₃.

### Example 2

Calcitriol was diluted 1000-fold with ethanol, and further diluted 100-fold with an ophthalmic oily base consisting of purified sesame oil to prepare an ophthalmic composition containing 0.5 µg/ml of calcitriol.

### Example 3

In 10 ml of the ophthalmic composition prepared by the procedures of Example 1 was dissolved 60 mg of vitamin C (L-ascorbic acid-phosphate) to give an ophthalmic composition consisting of a mixed solution containing 6 mg/ml of vitamin C and 0.5 µg/ml of active vitamin D₃.

### Test example 1

Calcitriol was dissolved in a base consisting of sodium phosphate, sodium chloride, ethanol and Polysorbate 80 to prepare three solutions at a concentration of 1.0 x 10⁻⁶ M, 1.0 x 10⁻⁷ M and 1.0 x 10⁻⁸ M. Separately, a steroid, dexamethasone sodium was dissolved in the same base to prepare a 0.1% solution.

Each of 30 BALB/c mice got 2 stitches of 10-0 nylon thread in the central region of the cornea of one eye and then was left for migration of Langerhans cells in the corneas thereof. The mice were divided into 6 groups A-F each consisting of 5 animals, in which group A received no instillation, group B received the steroid (St), groups C-E received calcitriol at the three concentrations mentioned above, and group F received the base alone.

The instillation was started at a frequency of three times per day on the day following stitching. Fourteen days after stitching, the mice were killed and their eyes were extracted to assess the average density of Langerhans cells in the central corneal epithelium by immunostaining for each group. The results are shown in Table 1.

**Table 1**

| Density of Langerhans cells in the central corneal epithelium | | |
|---|---|---|
| Group | Density (cells/mm²) | p value |
| A (no instillation) | 5.0 ± 0.9 | |
| B (St) | 1.0 ± 0.3 | < 0.005 |
| C (1.0 x10⁻⁶ M) | 3.3 ± 0.6 | N.S. |
| D (1.0 x10⁻⁷ M) | 2.5 ± 0.7 | < 0.05 |
| E (1.0 x10⁻⁸ M) | 2.6 ± 0.5 | < 0.025 |
| F (base) | 4.6 ± 0.8 | |

Table 1 shows that the number of Langerhans cells is significantly reduced in groups C-E, which received calcitriol, as compared with group A, which received no instillation, and group F, which received the base alone. In addition, the calcitriol-induced reduction is concentration-independent. This suggests that calcitriol may have an optimal concentration for the inhibition of Langerhans cell migration. Generally, many migration inhibitors for cells involved in immunoreaction have such an optimal concentration.

During this instillation, eyes of the mice were also observed under a slit lamp microscope every second day to evaluate neovascularization as follows. The length of neovascularization in each of four quadrants of the cornea of each mouse is scored according to the following standard. The scores of neovascularization in four quadrants of each cornea are totalyzed (scores 0-12), and an average of the totalyzed scores for each group is calculated.
Score 0: No neovascularization is observed;
Score 1: Neovascularization does not reach the central region of the cornea (within a 1 mm radius from the center);
Score 2: Neovascularization reaches the central region of the cornea (within a 1 mm radius from the center) but does not reach the center;
Score 3: Neovascularization reaches the central cornea.

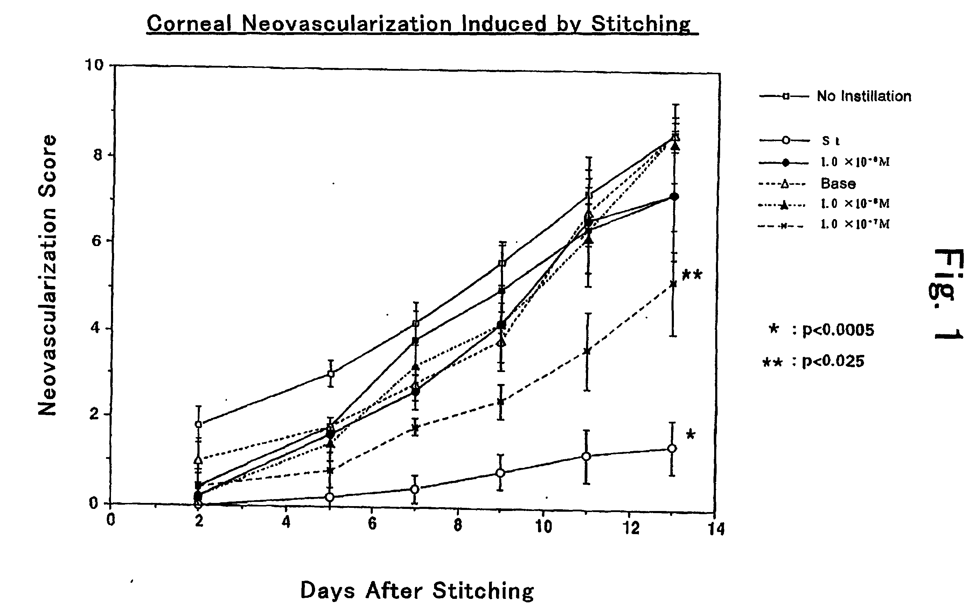

The results are shown in Fig. 1, which demonstrates that calcitriol also has an inhibitory effect on neovascularization.

### Test example 2

### Experiments:

To investigate the mechanism of the inhibitory effect of calcitriol (1α,25(OH)₂D₃) on Langerhans cell migration or neovascularization in the corneal epithelium, human corneal epithelial cells were grown in the presence of 1α,25(OH)₂D₃, after which the number of cells was determined and cytokine production level in the supernatant of the culture medium was also determined.

Human corneal epithelial cells kindly given by Dr. Sasaki (Toyonaka City Hospital, Osaka) were grown in Dulbecco's modified Eagle's medium (DMEM) /F-12 (1/1) supplemented with 10% fetal bovine serum, 5 µg/ml insulin, 0.1 µg/ml cholera toxin, 10 ng/ml human EGF, 0.5% DMSO and 40 µg/ml gentamicin at a temperature of 37°C and a humidity of 95% under 5% carbon dioxide. The medium was changed twice a week.

When human corneal epithelial cells were confluent, the medium was replaced with serum-free DMEM supplemented with 5 µg/ml insulin, 5 mg/ml transferrin and 5 ng/ml sodium selenite. After a 48 hour incubation, cells were seeded on a 6-well plate at 4 x 10⁶ cells per well and incubated in the presence of 0.1% 1α,25(OH)₂D₃ in ethanol at concentrations of 1.0 x 10⁻⁷ M, 1.0 x 10⁻¹¹ M and 1.0 x 10⁻¹⁵ M each for two wells. These wells were divided into two groups of 3 wells having different 1α,25(OH)₂D₃ concentrations, and the number of cells was determined after a 6 hour incubation for one group and a 12 hour incubation for the other group. Then, the culture media were each centrifuged at 150 g for 5 minutes and the supernatant was stored at -80°C. These experiments were performed at least in triplicate.

As a control, the same experiments were performed but in the absence of 1α,25(OH)₂D₃ (untreated) or in the presence of ethanol without 1α,25(OH)₂D₃ (solvent).

Cytokine quantitation was performed for interleukin-1α (IL-1α), interleukin-1β (IL-1β) and interleukin-8 (IL-8) using an ELISA kit (R & D System Corp.). The quantitation limits for the cytokines were 0.5 pg/ml for IL-1α, 1 pg/ml for IL-1β, and 10 pg/ml for IL-8. Calorimetry was performed at 450 nm using EL 308 (Bio-tek Instrument, Wincoski). The results of measurement of the three experiments were assembled and statistically analyzed by unpaired t-test.

### Results:

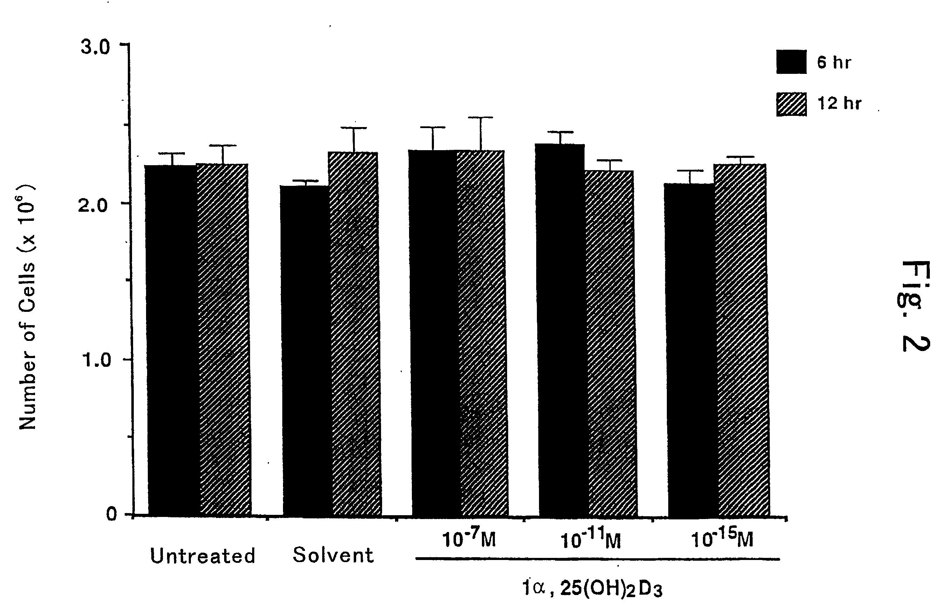

As shown in Fig. 2, no significant difference is found in cell growth between the groups treated with 1α,25(OH)₂D₃ and the solvent control group. Therefore, the *in vitro* influence of 1α,25(OH)₂D₃ on the growth of corneal epithelial cells is very weak.

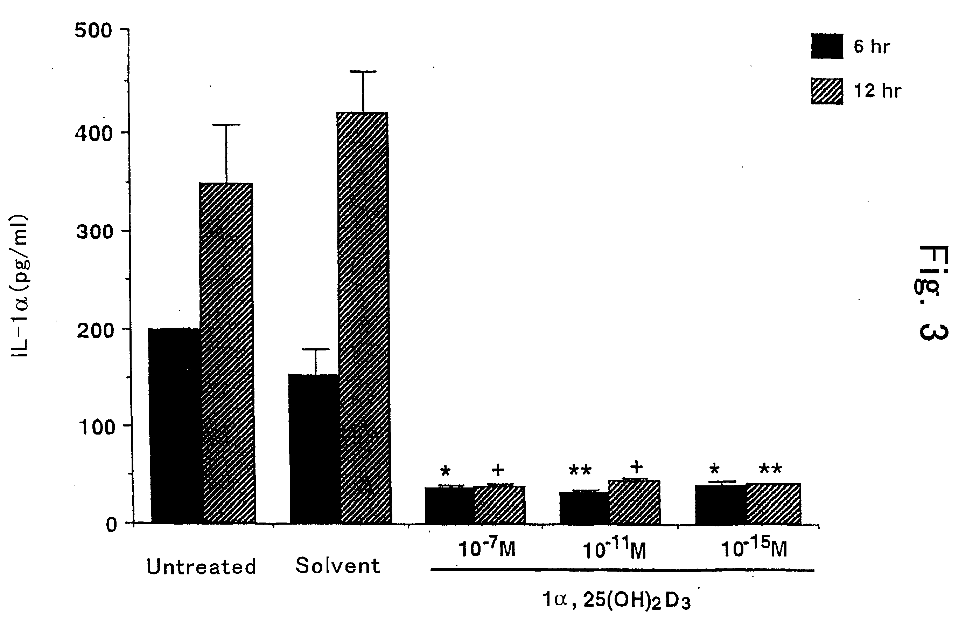

On the other hand, 1α,25(OH)₂D₃ exhibits an inhibitory effect on production of all of the cytokines IL-1α, IL-1β and IL-8, as shown in Figs. 3 to 5. The effect on IL-1α and IL-1β is especially pronounced. However, the effect on IL-8 is rather poor.

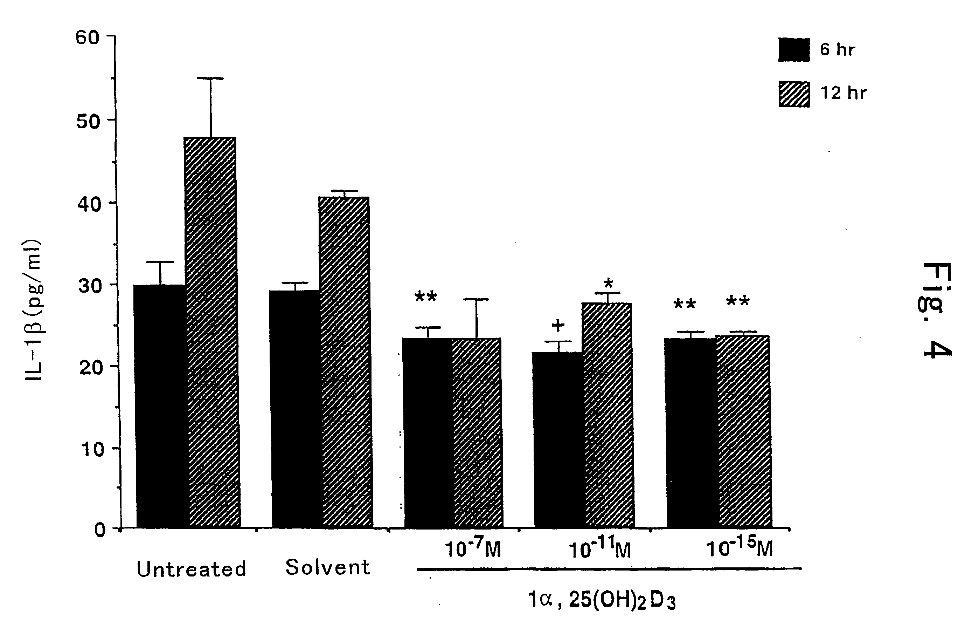

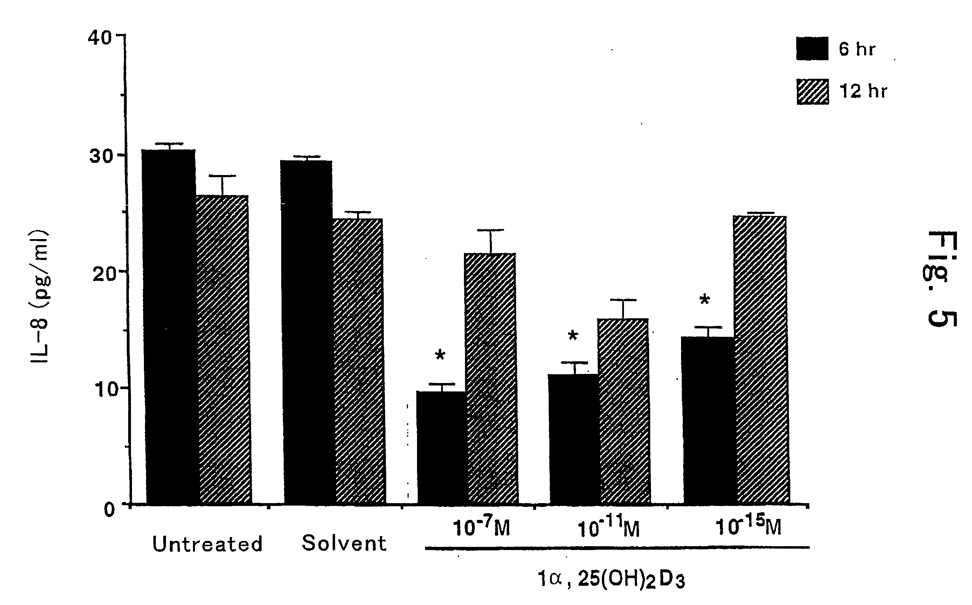

Specifically, Figs. 3 and 4 show that the amounts of IL-1α and IL-1β produced in the absence of 1α,25(OH)₂D₃ after a 6 hour incubation are 199.8 ± 0.65 pg/10⁶ cells and 29.9 ± 2.8 pg/10⁶ cells, respectively, and both increase to about 1.5 times after a 12 hour incubation, but the amounts of IL-1α and IL-1β produced after the 6 hour incubation in the presence of 1α,25(OH)₂D₃ are only 25% and about 77% as compared with the untreated group irrespective of the concentration of 1α,25(OH)₂D₃. These amounts are shown to scarcely increase even after the 12 hour incubation. Fig. 5 shows that the amount of IL-8 produced in the groups treated with 1α,25(OH)₂D₃ significantly decrease concentration-dependently after a 6 hour incubation as compared with the untreated group, but does not significantly decrease after a 12 hour incubation as compared with the untreated group.

These results show that 1α,25(OH)₂D₃ has a very strong *in vitro* inhibitory effect on IL-1 production in human corneal epithelial cells, but its inhibitory effect on IL-8 production is relatively weak. This is the first finding demonstrating that 1α,25(OH)₂D₃ directly inhibits IL-1 production in corneal epithelial cells. These results indicate that the inhibition of Langerhans cell migration in the corneal epithelium by 1α,25(OH)₂D₃ results from the inhibition of IL-1 production in the corneal epithelium by 1α,25(OH)₂D₃, because strong *in vitro* inhibitory effects on IL-1 production correspond to strong *in vivo* inhibitory effects on Langerhans cell migration and relatively weak *in vitro* inhibitory effects on IL-8 production correspond to relatively weak *in vivo* inhibitory effects on neovascularization.

## Claims

1. A Langerhans cell migration inhibitor comprising active vitamin D as an active ingredient.

2. The Langerhans cell migration inhibitor of Claim 1 wherein the active vitamin D is vitamin D₃ or a derivative or analog of vitamin D₃.

3. The Langerhans cell migration inhibitor of Claim 2 wherein the active vitamin D is vitamin D₃.

4. The Langerhans cell migration inhibitor of Claim 3 wherein the active vitamin D₃ is calcitriol or 22-oxacalcitriol.

5. The Langerhans cell migration inhibitor of any one of Claims 1 to 4 wherein the inhibitor is in the form of an ophthalmic solution.

6. The Langerhans cell migration inhibitor of Claim 5 wherein the inhibitor is used for the prevention and/or treatment of ocular inflammation.

7. The Langerhans cell migration inhibitor of Claim 6 wherein the ocular inflammation is keratoconjunctivitis.

8. The Langerhans cell migration inhibitor of Claim 5 wherein the inhibitor is used for the prevention of ocular inflammation.

9. The Langerhans cell migration inhibitor of Claim 8 wherein the ocular inflammation is phlyctenular keratitis or corneal infiltration.

10. The Langerhans cell migration inhibitor of Claim 5 wherein the inhibitor prevents and/or treats an ocular inflammation by inhibiting the production of interleukin-1 in corneal epithelial cells.
